Europäisches Patentamt

**European Patent Office**

Office européen des brevets

(11) Publication number: **0 221 642**

A2

(12) **EUROPEAN PATENT APPLICATION**

(21) Application number: **86306638.7**

(22) Date of filing: **28.08.86**

(51) Int. Cl.⁴: **G 01 N 21/55**
**G 01 N 33/12**

(30) Priority: **04.09.85 CA 489982**

(43) Date of publication of application:
**13.05.87 Bulletin 87/20**

(84) Designated Contracting States:
**BE CH DE FR GB LI NL SE**

(71) Applicant: **WESTINGHOUSE CANADA INC.**
**Box 510**
**Hamilton Ontario L8N 3K2(CA)**

(71) Applicant: **INSTRUMAR LIMITED**
**P.O. Box 13246 Station A**
**St John's Newfoundland A1B 4A5(CA)**

(72) Inventor: **Allan, Alasiair**
**PO Box 13246,Station A**
**St John's,Newfoundland A1B 4A5(CA)**

(72) Inventor: **Butt, Kenneth A.**
**29 Glenview Terrace**
**St.John's A1E 3H8 Newfoundland(CA)**

(72) Inventor: **Reimer, Ernest M.**
**1 Atlantic Avenue**
**St.John's A1E 1K9 Newfoundland(CA)**

(74) Representative: **van Berlyn, Ronald Gilbert**
**23, Centre Heights**
**London, NW3 6JG(GB)**

(54) **Fish color grader.**

(57) A hand held apparatus is disclosed to color grade fish fillets. The apparatus illuminates a sample fillet and measures the intensity of the resultant reflected light at certain frequencies at which hemoglobin displays peculiar absorption characteristics. The apparatus ratios these measured intensities and compares the ratios with stored sets of values representing ratios of intensities of reflection at these frequencies which would be expected for flesh of various color grades. The apparatus displays the color grade associated with the closest set of values.

CW-1108

## FISH COLOR GRADER

0221642

This invention relates to an apparatus to determine the color grade of a sample of a known substance. It has particular application to the grading of fish flesh.

In the commercial fishing industry, it is common to grade fish fillets in order to determine their acceptability and price. Of key concern is the concentration of hemoglobin in the fillet since its presence could result in an accelerated deterioration of the flesh. As hemoglobin is usually the dominant colorant in a fillet, color grading is employed to obtain an indication of the concentration of hemoglobin. Visual color grading is common and it is known to use standardized color chips to assist in such grading. However, the accuracy of visual comparisons between color chips and the fillet is limited by the phenomenon of metamerism. That is, two objects with different spectral reflectance curves may color match for some observers and not for others due to differences in color vision from person to person. Further, such two objects may color match for one observer under some lighting conditions and not under others. The accuracy of visual comparisons in the color grading of whitefish is also limited due to the necessity of discriminating between subtle differences in 20 shades of white. Thus, particularly for dockside fish fillet grading where lighting conditions can vary

pat012/12/2

**0221642**

considerably, an objective color instrument would eliminate color grade uncertainties.

Briefly stated, the present invention is an apparatus to measure the color grade of a sample of flesh of a known species comprising: a light source for illuminating the sample; means to detect the intensity of light reflected from the sample in response to illumination from the light source at a plurality of frequencies at which hemoglobin displays peculiar light absorption characteristics in order to obtain a set of intensities; means to compare the detected set of intensities with other sets of values, each other set of values representing the relative intensity of light at said frequencies corresponding to a known color grade of flesh of the species, in order to determine the color grade of the sample.

In the figures which illustrate a preferred embodiment of the invention:

Figure 1 is a graph showing the spectral absorption curve of hemoglobin;

Figure 2 is a schematic diagram of the components of the fish color grader of this invention; and

pat012/12/3

Figure 3 is a flow chart for the operation of the fish color grader of Figure 2.

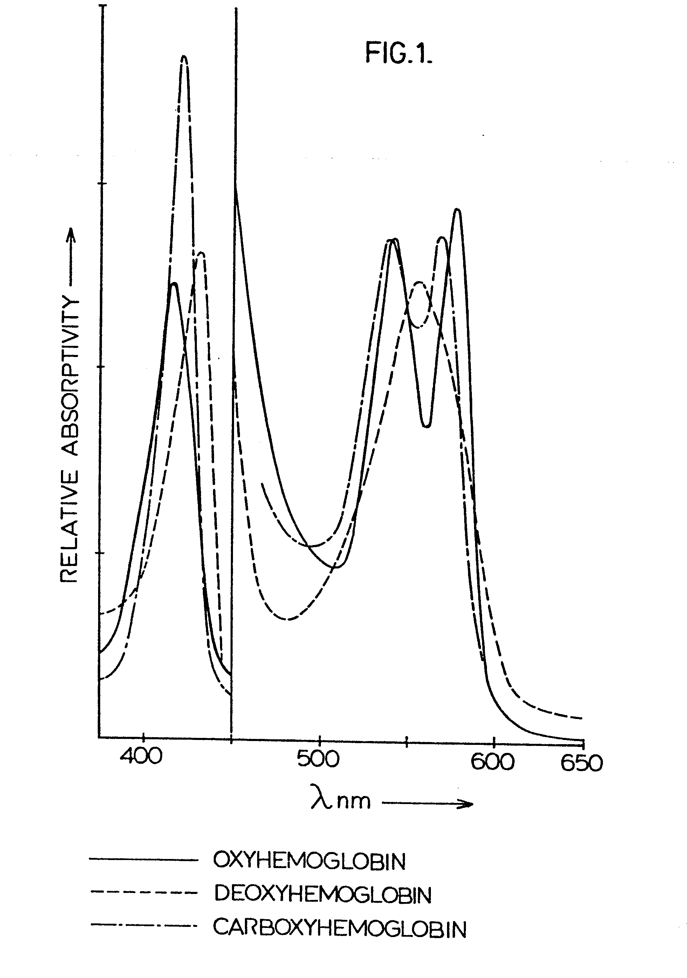

By reference to figure 1, it is seen that the components of hemoglobin have characteristics light absorption curves. The composite of these curves display significant local light absorption maxima centered at about the frequencies 425 nanometers and in the range of 540-580 light at about 625 nanometers. Consequently, where hemoglobin is the primary colorant in a particular species of flesh, it is possible to obtain a ratio of the intensity of reflected light at 625 nanometers to reflected light at 540-580 nanometers (or 425 nanometers) from various samples of this flesh and these ratios would indicate the relative concentrations of hemoglobin in the samples. Both the ratios of light at 625 nm to light at 540-580 nm and light at 625 nm to light at 425 nm may be utilized when increased sensitivity is desired.

In the present invention, to utilize the foregoing, the color grades of a number of samples of flesh of a particular species may be carefully determined by conventional means in a controlled setting. The aforedescribed ratios, which are indicative of hemoglobin concentration, may then be determined for each sample so that a table may be drawn up listing range of ratios and the particular color grade associated therewith. Such a table is incorporated in the apparatus of this invention so that after

pat012/12/4

0221642

determining the aforedescribed ratio for a sample of flesh of the particular species, the color grade of the sample may be readily determined.

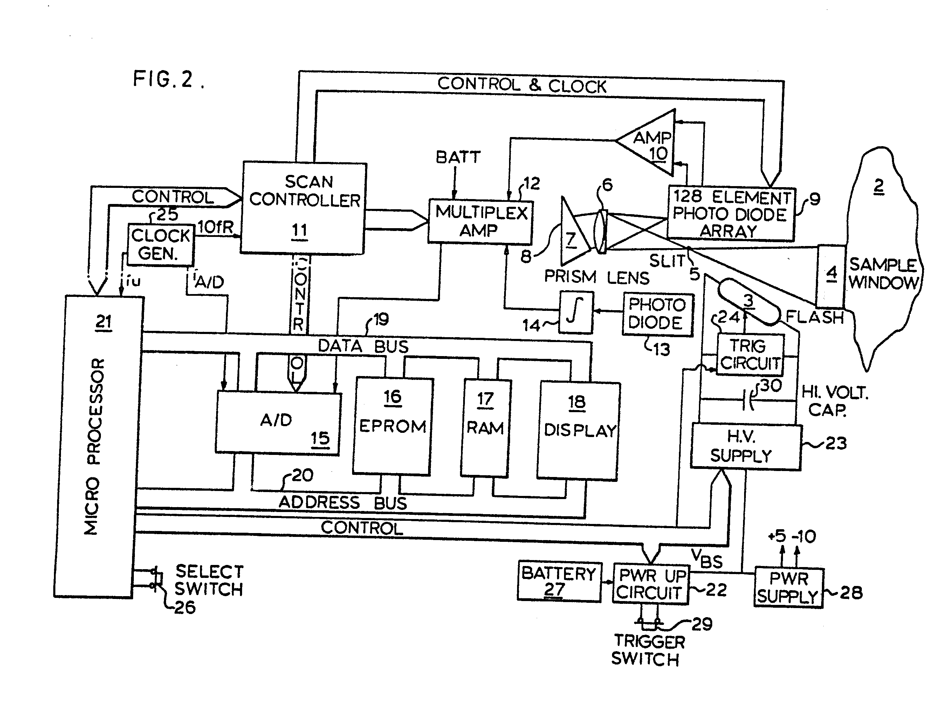

Figure 2 depicts the apparatus of this invention for measuring the color grade of a fish fillet 2 of given species, say, for example, a flounder fillet. The apparatus includes a high intensity pulsed light source 3 whose bandwidth covers the spectrum from 400 nm to 700 nm. A sample window 4 permits light from the light source 3 to impinge upon fish fillet 2 and it also permits light reflected from the fillet to reach a 1500 μ by 50 μ slit 5. The slit is positioned in front of and off the axis of a 35 millimetre focal length lens 6 behind which is positioned a Littrow mount prism 7 with a mirrored back surface 8. A photodiode array 9 is also positioned in front of and off the axis of lens 6 but on the side of the axis opposite to slit 5. The array 9 comprises 128 silicon photodiodes placed in side-by-side relation. The photodiodes of the array are selectively electrically coupled to amplifier 10 by scan controller 11. The output of the amplifier 10 is connected to an input of multiplex amplifier 12. Photodiode 13, which is in direct optical communication with light source 3, is connected to another input of multiplex amplifier 12. Multiplex amplifier 12 is electrically coupled to high speed (less than 30 microseconds), high resolution (at least 12 bits) A/D converter 15 under control of scan controller 11. The A/D converter, an

pat012/12/5

0221642

EPROM 16, a RAM 17, and a conventional LCD display 18 are connected between a data bus 19 and an address bus 20. The A/D converter outputs digital data to the data bus addressed to the RAM 17 under control of scan controller 11.

Overall system control is provided by microprocessor 21. More specifically, the microprocessor is connected to the data bus 19 and address bus 20. It is also connected to scan controller 11 and to a power up circuit 22, high voltage supply 23 and trigger circuit 24.

The microprocessor, and as well the scan controller and A/D converter, receive clock pulses from clock generator 25. The microprocessor receives an on/off indication from select switch 26.

The system is powered by a battery 27 which is connected to the power up circuit 22 and multiplex amplifier 12. The battery may be recharged through power supply 28. Power up circuit 22 is also connected to trigger switch 29 and high voltage supply 23. The high voltage supply is connected in parallel to a high voltage capacitor 30, trigger circuit 24, and the light source 3.

In operation, light source 3 is pulsed under control of microprocessor 21 after trigger switch 29 is activated. If the operator has placed the sample window 4 of the apparatus on a

pat012/12/6

0221642

fish fillet 2 prior to activating the trigger switch, then light from source 3 passing through the sample window will impinge upon fillet 2. As described hereinbefore, the amount of incident light which will be absorbed by a fillet at certain frequencies depends upon the concentration of hemoglobin in the fillet. Consequently, too, the light reflected from the fillet at these frequencies depends upon the fillet's concentration of hemoglobin. The light reflected from the fillet which passes through slit 5 refracts into a non-linear spectrum of from 400 nanometers (blue) at one end to 700 nanometers (red) at the other, as this is the wavelength range produced by light source 3. The spectrum hits lens 6 which directs it to prism 7 and the light entering prism 7 is reflected back from the mirrored back surface 8 of the prism to lens 6 where it is directed to the photodiode array 9. The 128 linearly arranged diodes of the array 9 each pick up a small portion of the spectrum of reflected light, to wit, light covering about 6 nanometers in red end and about 1.5 nanometers in the blue end. The 50 µ slit limits the resolution to about 12 nanometers in the red and about 3 nanometers in the blue end.

Scan controller 11, clocked at high speed by clock generator 25, couples the electrical output of selected diodes of the array through amplifier 10 to multiplex amplifier 12. The diodes selected are determined by microprocessor 21 which reads the light frequencies of concern from EPROM 16. The EPROM has been preprogrammed with information enabling the apparatus to color grade a specific species of meat, in this description, cod flesh. The EPROM, therefore, stores the information that the light frequencies of interest are 425 nanometers, 540-580 nanometers, and 625 nanometers.

The output of diode 13, which is in direct optical communication with light source 3, is also input to multiplex amplifier 12 after being integrated by integrator circuit 14. The multiplex amplifier multiplexes, in a time division manner, the signal generated by the diode 13 and the signals from all except one of diodes of array 9 coupled to the multiplex amplifier by scan controller 11. The multiplexed analog signals are accepted by A/D converter 15 under timing control of scan controller 11 and the resulting digital signals are stored in RAM 17. In this way, a set of multiplexed signals is stored in RAM 17, one signal for each diode in the array 9 and one signal for the output of diode 13 at the time of sampling of the diodes in the array.

Scan controller 11 sends an indication signal to the processor after it has prompted the A/D converter 15 to accept the last signal from multiplex amplifier 12. The processor then retrieves each data representation of the output from selected diodes of array 9 in turn and divides same by the stored representation of the output of diode 13 at the time of sampling. The set of ratioed output signals thus obtained represents the relative

pat012/12/8

0221642

intensity of reflected light from each sampled part of the spectrum irrespective of the intensity of light from source 3 which illuminated the fillet 2 at the time of sampling. The processor stores the ratioed measured set in RAM 17.

The processor repeats this sequence four times then averages the four measured and ratioed sets. Next, the processor divides the average reading at 625 nanometers by the average reading at 425 nanometers to obtain a ratio indicative of the color grade. The processor also determines the ratio between average intensity at 625 nanometers and in the range of 540 to 580 nanometers. The processor then compares these two ratios with ratios stored in EPROM 16 in search of the stored ratios that most closely approximate the measured color grade ratios. The ratios stored in EPROM 15 represent the relative intensity of light at 625 to 425 nanometers and at 625 to 540-580 nanometers which would be expected for a cod fillet of various color grades. The EPROM stores an indication of the grade associated with each set of ratios. The frequencies at which the EPROM ratios are based are also stored in the EPROM for supply to the processor for its determination of which channels will be processed and which of those remaining will be discarded. After the processor finds the closest stored set of values, it instructs the display to show the grade associated with the stored set, for example, "A" for premium, "B" for standard, "C" for a fillet which should be rejected.

pat012/12/9

The control sequence for the apparatus will now be described in detail with reference to both figures 1 and 2 of the drawings.

The battery 27 is electrically connected to the apparatus through a switch (not shown) to power up the apparatus (box 100). At this point the microprocessor 21 initialises the system (box 101). The initialization process includes self diagnostics on the power supply system memory, data bus, input/output port and timer and sets up the necessary counts and registers if a fault is detected then a red LED is activated and the system may or may not be shut down depending upon the seriousness of the fault. Assuming no malfunction, then a yellow LED is turned on and control is passed to the user. Following initialization, the microprocessor will read the active species select data from a low power latch (box 135). The latch is kept active even during power down as long as the hand held unit is attached to the power supply. The default "first power-up" mode is "COD". The processor then signals the display 18 through address buss 20 to accept a data signal on bus 19 which results in the appearance of the selected species message "COD" (at first use) on the display box (102). The microprocessor then enables an interrupt and begins to decrement an internal counter in response to pulses from the clock generator 25 (box 103a - 103f inclusive). The microprocessor is programmed to power down the apparatus when this counter times out. The processor then checks whether the select switch is in the "on" position (box 104a). This switch is

pat012/12/10

0221642

activated by the operator when he wishes a previously measured color grade of the fish fillet displayed. If the switch is off, the processor then tests the battery to ensure it is operational (box 105a), if not, the processor immediately powers down the apparatus (box 106a). So long as the battery is operational, the processor next checks whether the trigger switch 29 is in the "on" position (box 107). This switch is activated by the operator when he wishes the apparatus to take a color grade reading. If this switch is also off, then the control sequence will return to box 104a and will again test for activation of the select switch 26.

If, in reaching box 107 in the control sequence, the trigger switch is found to be "on", the processor stops all timers (box 109a), instructs the display to display the word "SAMPLING" (box 110) and clears a flag called VALID thereby setting it to its logically false value (box 111). The flag VALID will be reset to its logically true value later in the control sequence if the data sampled by the processor appears to be valid.

The processor then sets a light flash counter to four (box 112) and prompts the charge circuit 22 to energize the high voltage supply 23 (box 113). The high voltage supply then builds up the requisite charge on capacitor 30. The processor continuously checks that the high voltage supply has generated the requisite high voltage (box 114), if not, the processor instructs the

pat012/12/11

0221642

display to display "NO. H.V." (box 115) and then sets the display time to 4 secs (box 108) and starts a display timer (boxes 116 a-e) clocked by clock generator 25 and returns to box 105a for a re-check of the battery.  Note that if the operator has released the trigger switch by this time, the processor will instruct the display to display the selected species name "COD" after the display timer times out (boxes 109b, 131 and 102).

Assuming the proper high voltage has been generated, the processor sends a signal to the trigger circuit 24 to prompt it to fire the pulsed light source 3.  As aforedescribed, this results in the storage in RAM 17 of a digital representation of the intensity of light, at selected frequencies, reflected from a sample covering window 4.  It also results in the digital storage of the analog output of diode 13 which measures the intensity of light source 3 (box 117).  At this point, the processor checks the digital signal representing the level of output of diode 13 against a preset minimum in order to determine whether light source 3 did indeed flash (box 118).  If not, it instructs the display to display "NO FLASH" (box 119), sets the display time to 4 secs (box 108) and starts the display timer (boxes 116 a-e) and re-checks the battery (box 105a). The control sequence then proceeds from box 105a as described hereinbefore.

Assuming the strobe did flash, the processor then divides the digital representation of the output of selected diodes of array

pat012/12/12

by the output of diode 13 and stores the result in RAM 17, as aforedescribed (box 120).

Next the processor integrates the first eight data channels (box 121) and if it is the first of the four samples (box 132), it will store that value in both the minimum and maximum memory registers (box 133). On the subsequent three samples, the values of the min/max registers are compared with the present sample value. Depending upon whether the sample value is greater than the maximum or less than the minimum it will replace the value whose condition previously presented is true, if any (box 134).

The processor then decrements the flash counter (box 122) and, if this counter has not decremented to zero, the processor again prompts the charge circuit (box 113), fires the flash, and stores the new measured set of values. After looping through this sequence four times, the flash counter reaches zero (box 123) and the processor then finds the difference between the maximum and minimum values previously obtained (box 124). If the measured difference value falls outside of the limit of permissible range values stored in the EPROM, the processor interprets the data as faulty (box 125), instructs the display to display "REPEAT" (box 126), sets the display timer and then re-checks the select switch, battery, and trigger switch through boxes 104a, 105a, 107 and a portion of the control sequence aforedescribed.

pat012/12/13

0221642

If the data falls within the present ranges, the processor averages the measured sets, obtains a ratio indicative of the color grade, corresponding with the selected species and searches for the closest ratio in EPROM 16, all as aforedescribed.

After the processor finds the closest stored set of values, it sets the VALID flag to true (box 128) and, instructs the display to show the grade associated with the stored set (box 130). The processor then sets the display time to 20 seconds (box 131) and starts the display timer (box 116 a-e) and checks the select switch (box 104a), the battery (box 105a), and trigger switch (box 107) as previously discussed. If the trigger switch has been released, the determined grade will be displayed until the display timer times out, after which the species message "COD" will again be displayed on the display (boxes 109b, 135, 102).

The purpose of the select switch 26 is to redisplay a measured grade after it has ceased to be displayed in consequence of the display timer timing out. Thus, when the control sequence reaches box 104a, if the select switch is activated, but not held for longer than two seconds (box 132, and 133 a-e) then control passes to box 129, and, if the flag VALID has been set, the previously measured grade is displayed (box 130) and the display timer restarted (boxes 131 and 116 a-e). If the flag VALID is not set, which is the case prior to the first grade determination has been aborted due to faulty data, then activation of the

pat012/12/14

select switch merely results in the display of the current species name i.e.:  "COD" (box 129).

However, if the select switch 26 is held down for longer then momentary (i.e. greater than 2 seconds) (box 104b) then the microprocessor enters a loop where both the battery level and select switch status are checked until either a two second timer times out and updates the species latch with the next species (boxes 133 and 134) or the battery fails and the system powers down (boxes 105b and 106b) or the select switch is released causing the flow to resume with box 129.

EPROM 16 may be re-programmed with data which allows the apparatus to color grade other species of fish.  The apparatus of this invention, when set up for grading whitefish, is calibrated against a barium sulfate white reference surface and correction factors based on this reference color are determined automatically by the microprocessor for correction of each color channel during measurement.

The apparatus, as described, may be made so as to be watertight and hand held and may be fabricated with relatively inexpensive components.  The window 4 should be fabricated so as to be easily cleanable and scratch resistant.

**0221642**

While a preferred embodiment has been described, numerous variations are possible which fall within the spirit of this invention. For example, the monochromator may be replaced by a holographic grating or wedge filter; the light source could illuminate the fillet via a fibre optic bundle rather than directly, and the apparatus could be configured to function at a set stand-off from the fillet rather than by window 4 contacting the fillet 2.

The device can be adapted to grade other types of meat. Similar processing strategies would be utilized to establish relative hemoglobin levels. However, the large concentration of heme proteins remaining in bled meats would require that these strategies examine additional frequencies in the nominal color spectrum from 400 to 700 nanometers in wavelength.

This application has been specifically directed toward the color grading of whitefish, and it will become apparent to those skilled in the art that a similar device may be effectively utilized in the color grading of tuna and salmon. A further extension of the present device is in the grading of P.S.E. pork (pale, soft, exudative) wherein a light probe is inserted into the pork undergoing test to sample the pork flesh coloration below the surface. The device may also be used to color grade lumber when calibrated for this purpose.

pat012/12/16

0221642

THE EMBODIMENTS OF THE INVENTION IN WHICH AN EXCLUSIVE

PROPERTY OR PRIVILEGE IS CLAIMED ARE DEFINED AS FOLLOWS:

1.  An apparatus to measure the color grade of a sample of flesh of a known species comprising:

    a.  a light source for illuminating the sample;

    b.  means to detect the intensity of light reflected from the sample in response to illumination from the light source at a plurality of frequencies at which hemoglobin displays peculiar light absorption characteristics in order to obtain a set of intensities;

    c.  means to compare said detected set of intensities with other sets of values, each said other set of values representing the relative intensity of light at said frequencies corresponding to a known color grade of flesh of said species, in order to determine the color grade of said sample.

2.  The apparatus of claim 1 including means to adjust each said detected intensity in accordance with the intensity of said light source.

pat012/12/17

3. An apparatus to measure the color grade of a sample of flesh of a known species comprising:

a. a light source for illuminating the sample;

b. means to detect the intensity of light reflected from the sample in response to illumination from the light source at at least one first frequency where light is minimally absorbed by hemoglobin and at least one second frequency where light is absorbed a significant peculiar amount by hemoglobin;

c. means responsive to said at least one first frequency and said at least one second frequency to obtain at least one ratio;

d. means to compare said ratio with compatible ratios representing known color grades of flesh of said species in order to determine the color grade of the sample.

4. The apparatus of claim 3 wherein said at least one first frequency is at least one of about 425 nanometers and a frequency in the range of about 540 to 580 nanometers and said at least one second frequency is about 625 nanometers.

5. The apparatus of claim 4 wherein said flesh is whitefish flesh.

pat012/12/18

1/5

0221642

FIG.1.

RELATIVE ABSORPTIVITY →

λ nm →

———————— OXYHEMOGLOBIN

- - - - - - - DEOXYHEMOGLOBIN

—·—·—·— CARBOXYHEMOGLOBIN

FIG.2.

0221642

FIG.3A.

0221642

POWER UP ~100

INITIALIZE ~101

(B)

READ SPECIES LATCH ~135

DISPLAY SPECIES NAME ~102

SET POWER DOWN TIME TO 3 MIN ~103a

ENABLE POWER DOWN TIMER ~103b

(C)

POWER DOWN TIMER INTERRUPT ? ~103c — NO

YES

DECREMENT POWER DOWN TIME ~103d

POWER DOWN TIME =0 ? ~103e — NO

YES

CONTINUE ~103f

IS SELECT ON ? ~104a — YES → (A)

NO

IS BATTERY OK ? ~105a — NO

YES

POWER DOWN ~106a

IS TRIGGER ON ? ~107 — NO

YES → (D)

FIG.3B.

4/5

0221642

FIG.3C.

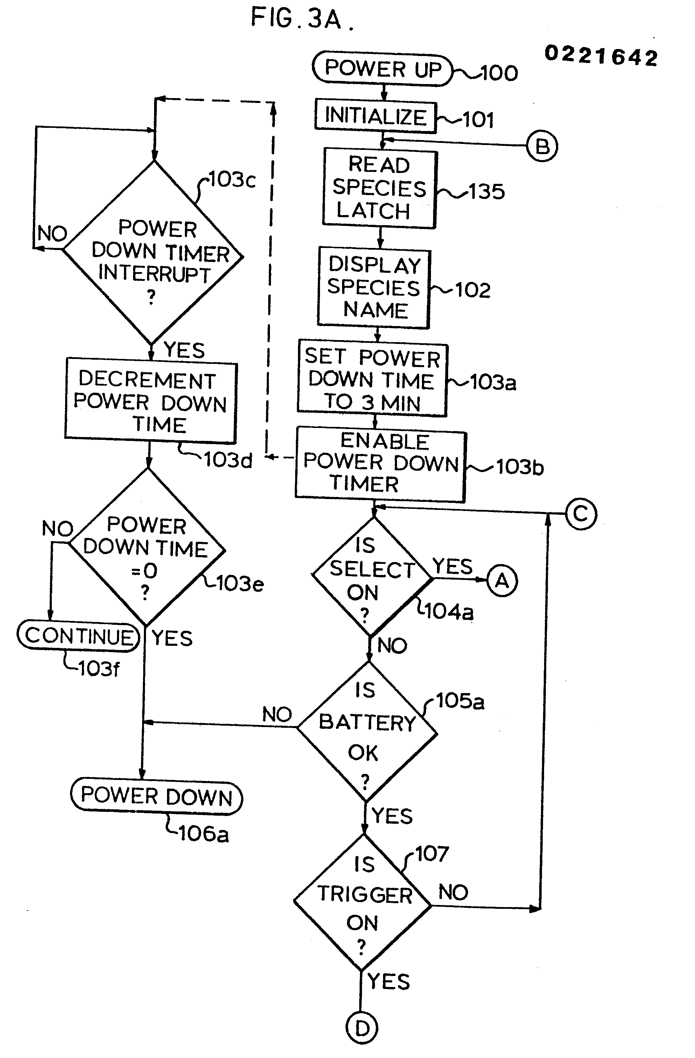

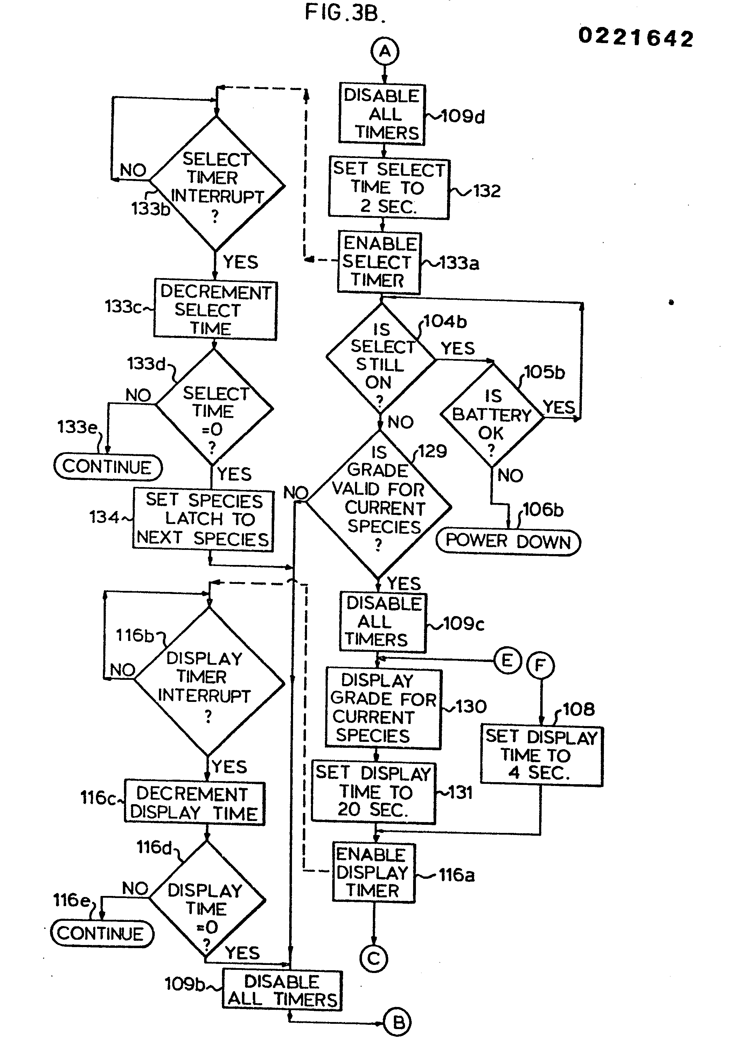

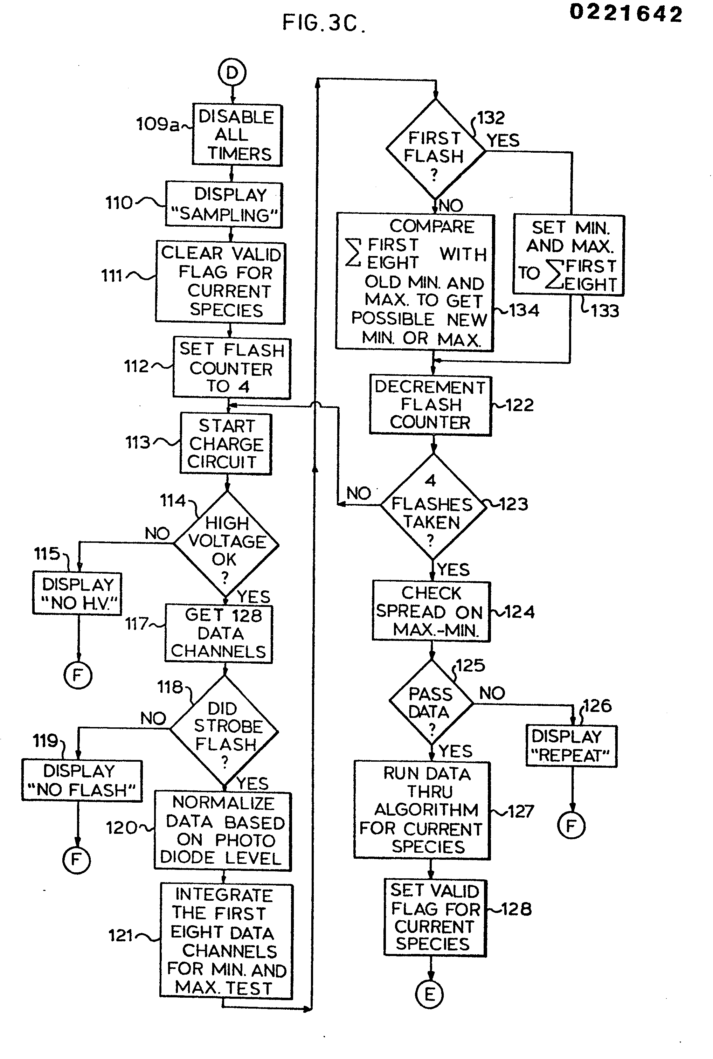

Flowchart (FIG.3C):

- (D) → **DISABLE ALL TIMERS** (109a)
- → **DISPLAY "SAMPLING"** (110)
- → **CLEAR VALID FLAG FOR CURRENT SPECIES** (111)
- → **SET FLASH COUNTER TO 4** (112)
- → **START CHARGE CIRCUIT** (113)
- → **HIGH VOLTAGE OK ?** (114)
  - NO → **DISPLAY "NO H.V."** (115) → (F)
  - YES → **GET 128 DATA CHANNELS** (117)
- → **DID STROBE FLASH ?** (118)
  - NO → **DISPLAY "NO FLASH"** (119) → (F)
  - YES → **NORMALIZE DATA BASED ON PHOTO DIODE LEVEL** (120)
- → **INTEGRATE THE FIRST EIGHT DATA CHANNELS FOR MIN. AND MAX. TEST** (121)
- → **FIRST FLASH ?** (132)
  - YES → **SET MIN. AND MAX. TO $\sum$ FIRST EIGHT** (133)
  - NO → **COMPARE $\sum$ FIRST EIGHT WITH OLD MIN. AND MAX. TO GET POSSIBLE NEW MIN. OR MAX.** (134)
- → **DECREMENT FLASH COUNTER** (122)
- → **4 FLASHES TAKEN ?** (123)
  - NO → (back to START CHARGE CIRCUIT)
  - YES → **CHECK SPREAD ON MAX.–MIN.** (124)
- → **PASS DATA ?** (125)
  - NO → **DISPLAY "REPEAT"** (126) → (F)
  - YES → **RUN DATA THRU ALGORITHM FOR CURRENT SPECIES** (127)
- → **SET VALID FLAG FOR CURRENT SPECIES** (128)
- → (E)